(19) Europäisches Patentamt
European Patent Office
Office européen des brevets

(11) Veröffentlichungsnummer: **0 397 601 A1**

(12) # EUROPÄISCHE PATENTANMELDUNG

(21) Anmeldenummer: 90810338.5

(51) Int. Cl.5: **C07D 213/64, A01N 47/34**

(22) Anmeldetag: 03.05.90

(30) Priorität: 11.05.89 CH 1776/89

(43) Veröffentlichungstag der Anmeldung: 14.11.90 Patentblatt 90/46

(84) Benannte Vertragsstaaten: AT BE CH DE DK ES FR GB IT LI NL

(71) Anmelder: CIBA-GEIGY AG
Klybeckstrasse 141
CH-4002 Basel(CH)

(72) Erfinder: Drabek, Jozef, Dr.
Benkenstrasse 12
CH-4104 Oberwil(CH)

(54) **Benzoylphenylharnstoffe.**

(57) Substituierte N-Benzoyl-N′-[2-fluor-4-(3-chlorpyridin-2-yloxy)-phenyl]-harnstoffe der Formel I

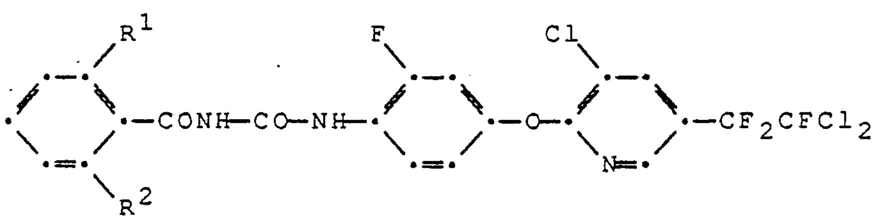

(I),

worin $R^1$ und $R^2$ beide Fluor oder $R^1$ Chlor und $R^2$ Wasserstoff bedeuten, sowie deren Salze; Verfahren und Zwischenprodukte zur Herstellung dieser Verbindungen sowie diese enthaltende Mittel zur Bekämpfung von Insekten, Vertretern der Ordnung Akarina und Schnecken, speziell von pflanzenschädigenden Insekten.

EP 0 397 601 A1

## Benzoylphenylharnstoffe

Die vorliegende Erfindung betrifft neue substituierte N-Benzoyl-N′-[2-fluor-4-(pyridin-2-yloxy)-phenyl]-harnstoffe mit insektizider und akarizider Wirkung, Verfahren zu ihrer Herstellung, sowie deren Verwendung zur Schädlingsbekämpfung, vorzugsweise in der Landwirtschaft.

Die neuen N-Benzoyl-N′-[4-(pyridin-2-yloxy)-phenyl]-harnstoffe entsprechen der Formel I

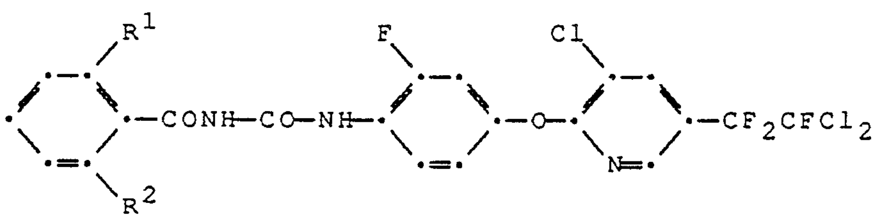
(I),

worin
$R^1$ und $R^2$ beide Fluor oder
$R^1$ Chlor und $R^2$ Wasserstoff bedeuten.

Die Erfindung wird durch die beiden Verbindungen N-(2,6-Difluorbenzoyl)-N′-{2-fluor-4-[3-chlor-5-(2,2-dichlor-1,1,2-trifluorethyl)-pyridin-2-yloxy]-phenyl}-harnstoff und N-(2-Chlorbenzoyl)-N′-{2-fluor-4-[3-chlor-5-(2,2-dichlor-1,1,2-trifluorethyl)-pyridin-2-yloxy]-phenyl}-harnstoff verkörpert.

Die Verbindungen der Formel I können analog an sich bekannter Verfahren hergestellt werden (siehe z.B. die deutschen Offenlegungsschriften No. 2 123 236, 2 601 760 oder 3 240 975).

So kann man die Verbindungen der Formel I erhalten, indem man das 2-Fluor-4-[3-chlor-5-(2,2-dichlor-1,1,2-trifluorethyl)-pyridin-2-yloxy]-anilin der Formel II

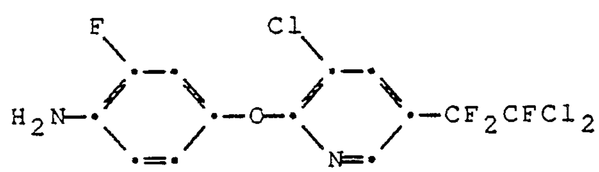
(II)

vorzugsweise in einem inerten organischen Lösungsmittel mit der äquimolaren Menge eines Benzoylisocyanates der Formel III umsetzt

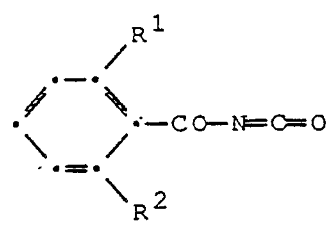
(III).

Die Verbindungen der Formel I können auch erhalten werden, indem man das Isocyanat der Formel IV

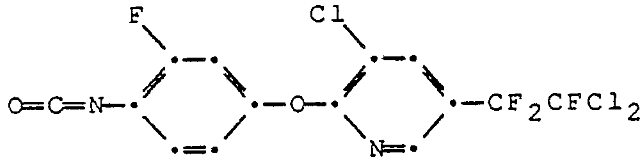
(IV)

vorzugsweise in einem inerten organischen Lösungsmittel mit der äquivalenten Menge eines Benzamides bzw. Urethans der Formel V umsetzt

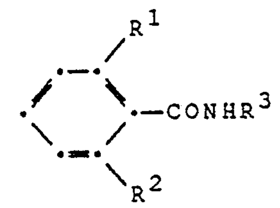

(V) .

In den obigen Formeln II bis V bedeuten $R^1$ und $R^2$ beide Fluor oder $R^1$ Chlor und $R^2$ Wasserstoff; $R^3$ steht für Wasserstoff oder einen Rest -$COOR^4$, wobei $R^4$ $C_1$-$C_6$-Alkyl, Phenyl oder Benzyl bedeutet.

Die erwähnten Verfahren werden vorzugsweise unter normalem Druck in Gegenwart eines organischen Lösungs- oder Verdünnungsmittels durchgeführt. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Die Verfahren werden im allgemeinen bei einer Temperatur von -10 bis +200 °C, vorzugsweise zwischen 0 und 100 °C, z.B. bei Raumtemperatur durchgeführt. Das erstgenannte Verfahren kann gegebenenfalls in Gegenwart einer organischen Base, z.B. Triäthylamin, stattfinden.

Die Ausgangsstoffe der Formeln III und V sind bekannt und können analog bekannter Verfahren hergestellt werden. Die Verbindung der Formel II kann in an sich bekannter Weise dadurch hergestellt werden, dass man 2-Fluor-4-hydroxyanilin in einem inerten organischen Lösungsmittel, in Gegenwart der mindestens äquimolaren Menge einer Base mit 2,3-Dichlor-5-(2,2-dichlor-1,1,2-trifluorethyl)-pyridin kondensiert entsprechend dem Schema:

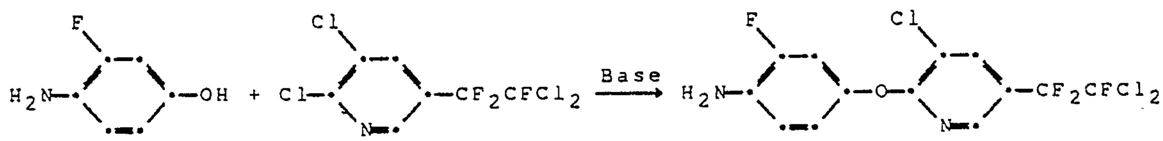


(II)

Zum 2-Fluor-4-[3-Chlor-5-(2,2-dichlor-1,1,2-trifluorethyl)-pyridin-2-yloxy]phenylisocyanat der Formel IV gelangt man, indem man das Anilin der Formel II nach allgemein üblichem Verfahren mit Phosgen behandelt. Das Anilin der Formel II und das Isocyanat der Formel IV sind neue Verbindungen und bilden ebenfalls einen Gegenstand der Erfindung. Die weiterhin als Ausgangsstoffe zu verwendenden Benzamide der Formel V sind bekannt oder können nach bekannten Verfahren dargestellt werden (vgl. z.B. Beilstein "Handbuch der organischen Chemie" Bd. 9, S. 336).

Zu den neuartigen Verbindungen der Formel I gehören erfindungsgemäss auch deren Salze, die sich nicht nur durch hohe insektizide Wirkung, sondern auch durch gute Löslichkeit in Lösungs- und Verdünnungsmitteln, insbesondere in organischen Lösungsmitteln, sowie durch verbesserte Formulierbarkeit auszeichnen.

Hervorzuheben sind die Metallsalze der erfindungsgemässen Verbindungen der Formel I, insbesondere ihre Alkali- und Erdalkalisalze, vorzugsweise die Natriumsalze und Kaliumsalze. Diese Salze werden in an sich bekannter Weise hergestellt, z.B. durch Umsetzen einer Verbindung der Formel I mit einem Metallalkanolat, wie Natriumäthylat oder Kaliummethylat. Bei einem vorgegebenen Salz können durch Umsalzen erwünschte Salze anderer Metalle erhalten werden.

Von besonderer Bedeutung sind die Salze der Verbindungen der Formel I mit organischen Basen, die im wesentlichen gekennzeichnet sind durch das Vorliegen eines quarternären Stickstoffatoms. Diese Salze entsprechen der Formel Ia

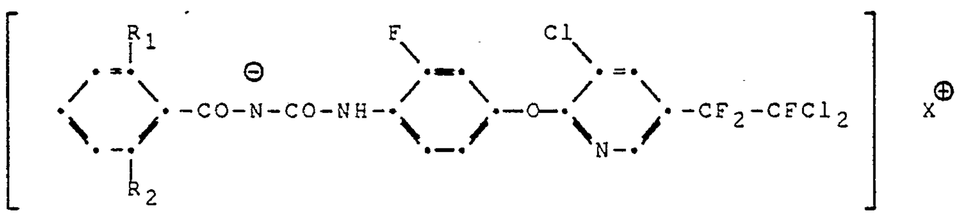 (Ia),

wobei $R^1$ und $R^2$ die oben gegebenen Bedeutungen besitzen und $X^{\oplus}$ das Kation einer organischen Base bedeutet. Vorzugsweise steht $X^{\oplus}$ für folgende organische Kationen:

$(CH_3)_4 N^{\oplus}$, $(C_2H_5)_4 N^{\oplus}$, $(n-C_3H_7)_4 N^{\oplus}$, $(i-C_3H_7)_4 N^{\oplus}$,

$(n-C_4H_9)_4 N^{\oplus}$, 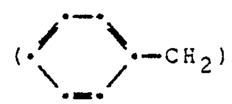 $(CH_3)_3 N^{\oplus}$,

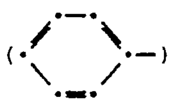 $(CH_3)_3 N^{\oplus}$, 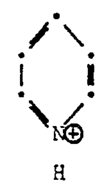 , $(C_4H_9)_3 NH^{\oplus}$,

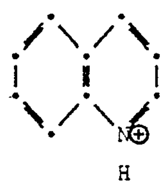 und $[CH_3-(CH_2)_n-]_3 N^{\oplus}-CH_3$,

wobei n eine Zahl 8 bis 12 bedeutet. Unter den Salzen gemäss Formel Ia) sind auch Gemische dieser Salze mit unterschiedlichen Kationen zu verstehen. Die Salze der For mel Ia können in an sich bekannter Weise hergestellt werden, z.B. durch Umsetzung einer Verbindung der Formel I mit entsprechenden Ammoniumhydroxyden der Formel $X^{\oplus}$ $(OH)^{\ominus}$, wobei $X^{\oplus}$ die vorstehend angegebene Bedeutung hat.

Es sind bereits neben den substituierten N-Benzoyl-N′-(4-pyridyloxyphenoxy)harnstoffen mit insektizider und fungizider Wirkung gemäss den offengelegten Japanischen Patentanmeldungen JA-A 62 195 395 und JA-A 63 122 661 auch N-Benzoyl-N′-2,5-dichlorphenyl-harnstoffe mit einem Halogenalkoxysubstituenten in 4-Stellung am Phenylring als Insektizide bekannt (vgl. US-Patentschrift Nr. 4.518.804 und deutsche Offenlegungsschrift Nr. 2848794). In der US-Patentschrift Nr. 4.162.330 werden ferner insektizid wirksame N-Halogenbenzoyl-N′-(3,5-dichlor-4-halogenalkenyloxy-phenyl)-harnstoffe und in der offengelegten Japanischen Patentanmeldung JA-A 63 270 662 wird der N-2,6-Difluorbenzoyl-N′-[2-fluor-4-(3-chlor-5-trifluormethylpyrid-2-yloxy)-phenyl]-harnstoff mit akarizider Wirkung beschrieben. Gegenüber den in diesen Schrifttumstellen beschriebenen Verbindungen weisen die erfindungsgemässen neuartigen Benzoylphenylharnstoffe der Formel I als wesentliches unterscheidendes Strukturmerkmal eine in 4-Stellung am Phenylring befindliche 3-Chlor-5-(2,2-dichlor-1,1,2-trifluorethyl)-pyridin-2-yloxy-Gruppe auf.

Ueberraschenderweise wurde nun gefunden, dass die vorliegenden Verbindungen der Formel I und ihre Salze bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Insekten und Vertretern der Ordnung Akarina. Erwähnenswert ist die ausgeprägte Blattpenetrationswirkung der erfindungsgemässen Verbindungen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, sowie von Vertretern der Ordnung Akarina der Familien: Ixodidae, Argasidae, Tetranychidae und Dermanyssidae.

Neben ihrer Wirkung gegenüber Fliegen, wie z.B. Musca domestica, und Mückenlarven können

Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens), sowie in Obst- und Gemüsekulturen (z.B. Laspeyresia pomonella, Leptinotarsa decemlineata und Epilachna varivestis) eingesetzt werden. Die Verbindungen der Formel I zeichnen sich durch eine ausgeprägte ovizide und vor allem larvizide Wirkung gegen Insekten, insbesondere Larven von fressenden Schadinsekten, aus. Werden Verbindungen der Formel I von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I eignen sich vorteilhafterweise auch zur Bekämpfung von pflanzenschädigenden Spinnmilben, wie z.B. den folgenden Obst- und Gemüsekulturen befallenden Milbenspezies: Tetranychus urticae, Tetranychus cinnabarinus, Panonychus ulmi, Broybia rubrioculus, Panonychus citri, Eriophyes piri, Eriophyes ribis, Eriophyes vitis, Tarsonemus pallidus, Phyllocoptes vitis und Phyllocoptruta oleivora.

Die Verbindungen der Formel I können ferner zur Bekämpfung von Ektoparasiten, wie Lucilia sericata, an Haus- und Nutztieren eingesetzt werden, z.B. durch Tier-, Stall- und Weidebehandlung.

Die erfindungsgemässen Benzoylharnstoffe der Formel I weisen ferner Eigenschaften auf, die ihren Einsatz zur Bekämpfung von Schnecken ermöglichen. Die repellierende und frasshemmende Wirkung dieser Verbindungen ist im Laborversuch häufig nur schwierig erkennbar. Im Freiland werden jedoch in landwirtschaftlichen und gärtnerischen Pflanzenkulturen auch bei sehr niedrigen Aufwandmengen gute Wirkungen beobachtet. Insbesondere werden empfindliche Salat-, Gemüse- und Obstkulturen (wie Erdbeerkulturen) sowie die Kulturen von Zierpflanzen und Blumen gegen Schneckenfrass geschützt. Die Wirksamkeit der erfindungsgemässen Verbindungen der Formel I erstreckt sich auf alle Nackt-und Gehäuseschnekken, welche in der Mehrzahl als polyphage Schädlinge landwirtschaftlicher, gärtnerischer und Zierpflanzen-Kulturen auftreten. Es kann sowohl frasshemmende als auch abtötende Wirkung eintreten. Zu den landlebenden Schnecken gehören einige besonders wichtige Schädlinge wie z.B. die Nacktschnecken Arion rufus (Grosse Wegschnecke); Arion ater und andere Arionidae, Limax-Arten und die Ackerschnecken, u.a. Deroceras reticulatum und D. agreste aus der Familie Limacidae, sowie Arten aus der Familie Milacidae. Weiterhin schädigen auch die Gehäuseschnecken u.a. der Gattungen Bradybaena, Cepaea, Cochlodina, Discus, Euomphalia, Galba, Helicigona, Helix, Helicella, Helicodiscus, Lymnaea, Opeas, Vallonia und Zonitoides, Nutzpflanzen im landwirtschaftlichen und gärtnerischen Bereich.

Die gute pestizide, insbesondere insektizide, Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der

physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss-oder Tallöl gewonnen werden können. Ferner sind als Tenside auch die Fettsäure-methyltaurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäure gruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, des Wirkstoffes der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Gewöhnlich beträgt die eingesetzte Aufwandmenge der erfindungsgemässen Wirkstoffe der Formel I - insbesondere für landwirtschaftliche Kulturflächen - 0,025 bis 1,0 kg/ha, vorzugsweise 0,1 bis 0,5 kg/ha, beispielsweise 0,1 bis 0,25 kg/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die folgenden Beispiele zeigen die Herstellung der erfindungsgemässen Verbindungen der Formel I sowie des als Zwischenprodukt benötigten Anilids der Formel II. Die Temperaturen sind in Centigrad gegeben.

Beispiel 1: Herstellung von N-(2,6-Difluorbenzoyl)-N′-{2-fluor-4- [3-chlor-5-(2,2-dichlor-1,1,2-trifluoräthyl)-pyridin-2-yloxy]-phenyl}-harnstoff

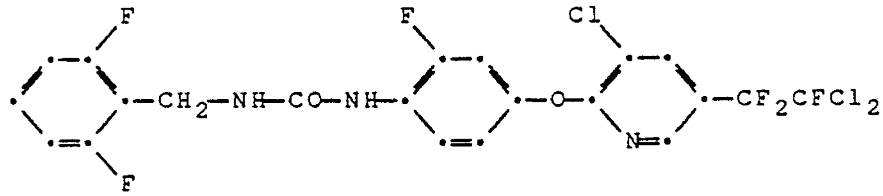

Zu einer Lösung von 4,67 g 2-Fluor-4-[3-chlor-5-(2,2-dichlor-1,1,2-trifluorethyl)-pyridin-2-yloxy]-anilin in 20 ml Toluol tropft man unter Rühren 2,19 g 2,6-Difluorbenzyloxyisocyanat. Das Reaktionsgemisch wird anschliessend 5 Stunden bei Raumtemperatur gerührt, filtriert und der Filterrückstand mit Toluol gewaschen und getrocknet. Man erhält so das Titelprodukt als farblose Kristalle, die bei 196 - 200° C schmelzen.

In analoger Weise wird der N-(2-Chlorbenzoyl)-N′-{2-fluor-4-[3-chlor-5-(2,2-dichlor-1,1,2-trifluorethyl)-pyridin-2-yloxy]-phenyl}-harnstoff hergestellt (Smp. 190 - 191° C).

Beispiel 2: Herstellung von 2-Fluor-4-[3-chlor-5-(2,2-dichlor-1,1,2-trifluorethyl)-pyridin-2-yloxy]-anilin (Zwischenprodukt)

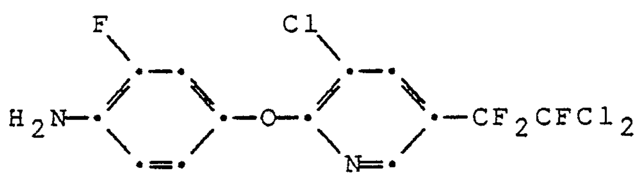

(II)

Zu einer Lösung von 10,17 g 2-Fluor-4-hydroxyanilin in 25 mi Dimethylsulfoxid gibt man 5,8 g pulverisiertes 85 %iges Kaliumhydroxid und rührt 20 Minuten. Dann tropft man 23,9 g 2,2-Dichlor-5-(2,2-dichlor-1,1,2-trifluorethyl)-pyridin dazu und rührt das Ganze während 10 Stunden bei Raumtemperatur. Dann verdünnt man das Reaktionsgemisch mit Wasser und extrahiert mit Methylenchlorid. Die organische Phase wird mit Wasser gewaschen, getrocknet und das Lösungsmittel abdestilliert. Das verbleibende Oel wird durch Chromatographie über eine mit Kieselgel (Merk 60) gefüllte Säule gereinigt. Als Fliessmittel dient Methylenchlorid. Nach Verdampfen des Lösungsmittels erhält man die Titelverbindung als farblose Kristalle vom Schmelzpunkt 61 - 62° C.

Beispiel 3: Formulierungen für Wirkstoffe der Formel I gemäss Beispiel 1 resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

| 4. Extruder-Granulat | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| 5. Umhüllungs-Granulat | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| 6. Suspensions-Konzentrat | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 4: Wirkung gegen Musca domestica:

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoff-konzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert. Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 5: Wirkung gegen Lucilia sericata:

Zu 9 ml eines Zuchtmediums wird bei 50 °C 1 ml einer 0,5 Gew.-% Aktivsubstanz enthaltenden wässrigen Zubereitung hinzugefügt. Nun werden ca. 30 frisch geschlüpfte Lucilia sericata-Larven zum Zuchtmedium gegeben, und nach 48 und 96 Stunden wird die insektizide Wirkung durch Ermittlung der Abtötungsrate festgestellt.

Die Verbindungen gemäss Beispiel 1 zeigen in diesem Test gute Wirkung gegen Lucilia sericata.

Beispiel 6: Wirkung gegen Aëdes aegypti:

Auf die Oberfläche von 150 ml Wasser, das sich in einem Behälter befindet, wird so viel einer 0,1 Gew.-%igen acetonischen Lösung des Wirkstoffes pipettiert, dass eine Konzentration von 800 ppm erhalten wird.

Nach Verdunsten des Acetons wird der Behälter mit 30 bis 40 2-tägigen Aëdes-Larven beschickt. Nach 1, 2 und 5 Tagen wird die Mortalität geprüft.

Die Verbindungen gemäss Beispiel 1 zeigen in diesem Test gute Wirkung gegen Aëdes aegypti.

Beispiel 7: Insektizide Frassgift-Wirkung:

Ca. 25 cm hohe eingetopfte Baumwollpflanzen werden mit wässrigen Wirkstoffemulsionen besprüht, die den Wirkstoff in Konzentrationen von 15 und 50 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24 °C und 60 % relativer Luftfeuchtigkeit durchgeführt. Nach 2 Tagen wird die %-Mortalität der Larven gegenüber unbehandelten Kontrollansätzen bestimmt.

Eine Wirkung von 80-100 % (Mortalität) zeigen die Verbindungen gemäss Beispiel 1 bei 15 ppm gegen Spodoptera-Larven und bei 50 ppm gegen Heliothis-Larven.

Beispiel 8: Wirkung gegen Epilachna varivestis:

Etwa 15-20 cm hohe Phaseolus vulgaris-Pflanzen (Buschbohnen) werden mit wässrigen, den zu prüfenden Wirkstoff in einer Konzentration von 800 ppm enthaltenden Emulsions-Zubereitungen besprüht. Nach dem Antrocknen des Sprühbelages werden pro Pflanze 5 Larven von Epilachna varivestis (Mexikanischer Bohnenkäfer) im 4. larvalen Stadium angesetzt. Ueber die infestierten Pflanzen wird ein Plastikzylinder gestülpt, der mit einem Kupfer-Gazedeckel abgedeckt ist. Der Versuch wird bei 28 °C und 60 % relativer Luftfeuchtigkeit durchgeführt.

Nach 2 und 3 Tagen wird die % Mortalität bestimmt. Zur Auswertung hinsichtlich allfälligem Frass-Schaden (Antifeeding-Effekt), Entwicklungs- und Häutungsstörungen werden die Versuchstiere während weiterer 3 Tage beobachtet.

Die Verbindungen gemäss Beispiel 1 zeigen gute Wirkung im obigen Test.

Beispiel 9: Ovizide Wirkung auf Heliothis virescens:

Entsprechende Mengenanteile einer benetzbaren pulverförmigen Formulierung, enthaltend 25 Gew.% des zu prüfenden Wirkstoffes, werden mit jeweils soviel Wasser vermischt, dass sich eine wässrige Emulsion mit einer Wirkstoffkonzentration von 800 ppm ergibt.

In diese wirkstoffhaltigen Emulsionen werden eintägige Eigelege von Heliothis auf Cellophan während drei Minuten eingetaucht und dann auf Rundfilter abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt.

Die Verbindungen gemäss Beispiel 1 zeigen gute Wirkung in obigem Test.

Beispiel 10: Wirkung auf Laspeyresia pomonella (Eier):

Abgelegte Eier von Laspeyresia pomonella, die nicht älter als 24 Stunden sind, werden auf Filterpapier für 1 Minute in eine acetonisch-wässrige Lösung, enthaltend 800 ppm des zu prüfenden Wirkstoffes, eingetaucht. Nach dem Antrocknen der Lösung werden die Eier in Petrischalen ausgelegt und bei einer Temperatur von 28 °C belassen. Nach 6 Tagen wird der prozentuale Schlupf aus den behandelten Eiern bewertet und die %-Mortalität bestimmt.

Die Verbindungen gemäss Beispiel 1 zeigen gute Wirkung in obigem Test.

Beispiel 11: Reproduktions-Beeinflussung von Anthonomus grandis:

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 400 ppm des zu prüfenden Wirkstoffes eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird die %-Mortalität der Eier bestimmt, d.h. wieviel Larven sich aus den deponierten Eiern entwickelt haben.

Zur Ermittlung der Dauer des die Reproduktion beeinflussenden Effektes der zu prüfenden Wirkstoffe

wird die Eiablage der Käfer noch weiter, d.h. während eines Zeitraums von etwa 4 Wochen überprüft. Die Bonitierung erfolgt anhand der Verminderung der Anzahl abgelegter Eier und daraus geschlüpfter Larven im Vergleich zu unbehandelten Kontrollen.

Die Verbindungen gemäss Beispiel 1 zeigen gute Wirkung im obigen Test.

Beispiel 12: Wirkung gegen Anthonomus grandis (Adulte)

Zwei eingetopfte Baumwollpflanzen im 6-Blattstadium werden jeweils mit einer wässrigen benetzungsfähigen Emulsions-Zubereitung enthaltend 400 ppm des zu prüfenden Wirkstoffes besprüht. Nach dem Antrocknen des Spritzbelages (nach etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die so behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Die Verbindungen gemäss Beispiel 1 zeigen gute Wirkung in obigem Test.

Beispiel 13: Insektizide Frassgift-Wirkung gegen Plutella xylostella

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 0,8 ppm enthalten und auf den Pflanzen antrocknen.

Nach zwei Tagen werden die behandelten Chinakohlpflanzen mit je 10 Plutella xylostella-Larven im zweiten larvalen Stadium besiedelt. Der Versuch wird bei 24°C und 60 % relativer Luftfeuchtigkeit im Dämmerlicht durchgeführt. Die Bonitur erfolgt nach 2 und 5 Tagen, es wird die %-Mortalität der Larven bestimmt.

Eine Wirkung von 100 % (Mortalität) zeigt die erste Verbindung des Beispiels 1 in diesem Test.

Beispiel 14: Frasshemmende Wirkung gegen Schnecken

Unter kontrollierten Versuchsbedingungen werden je 5 Nacktschnecken (Arion rufus) in einem Käfig mit 4 frischen Salatblättern über 17 Stunden belassen. Es handelt sich hierbei um Zwangsversuche, bei denen entweder nur durch eine Spritzapplikation behandelte oder unbehandelte Blätter angeboten werden. Die Wirkstoffkonzentration in der zur Applikation verwendeten wässrigen Zubereitung beträgt 0,5 Gew.-%. Das Ausmass des Frasses wird anhand von Gewichtsdifferenzen, Fotokopien des Frassbildes und nach augenscheinlichen Beurteilungskriterien gegenüber der unbehandelten Kontrolle ermittelt.

Daneben wird noch die allfällige Mortalität der Testtiere bestimmt.

Die Verbindungen gemäss Beispiel 1 zeigen gute Wirkung in obigem Test.

Beispiel 15: Akarizide Kontakt-Wirkung gegen Tetranychus urticae

Phaseolus vulgaris Pflanzen werden 12 Stunden vor dem Test auf akarizide Wirkung mit einem infestierten Blattstück aus einer Massenzucht von Tetranychus urticae belegt. Die übergelaufenen beweglichen Stadien werden aus einem Chromatographiezerstäuber mit den emulgierten Testpräparaten derart besprüht, dass kein Ablaufen der Spritzbrühe eintritt. Die jeweils verwendeten Emulsionszubereitungen weisen eine Wirkstoffkonzentration von 800 ppm auf. Nach zwei und 10 Tagen werden Larven, Adulte und Eier unter dem Binokular auf lebende und tote Individuen ausgewertet; das Ergebnis wird in Prozenten ausgedrückt.

Während der "Haltezeit" stehen die behandelten Pflanzen in Gewächshauskabinen bei 25°C.

Die Verbindungen gemäss Beispiel 1 zeigen im obigen Test gute Wirkung.

Beispiel 16: Ovizide Wirkung gegen Tetranychus urticae

Junge Bohnenpflanzen werden mit Weibchen von Tetranychus urticae besiedelt, die nach 24 Stunden wieder entfernt werden. Die mit Eiern besiedelten Pflanzen werden mit einer wässrigen Emulsions-Spritzbrühe, die 400 ppm des Wirkstoffes enthält, besprüht. Die Pflanzen werden anschliessend für 6 Tage bei 25° C inkubiert und danach ausgewertet. Aus dem Vergleich der Anzahl toter Eier, Larven und Adulten auf den behandelten zu denjenigen auf unbehandelten Vergleichspflanzen wird die prozentuale Reduktion der Population (% Wirkung) bestimmt.

Die Verbindungen der Formel I zeigen eine gute Wirkung gegen Tetranychus urticae in diesem Test.

Beispiel 17: Wirkung gegen Panonychus ulmi (OP und Carb. resistent)

Apfelsämlinge werden mit adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfennässe besprüht und im Gewächshaus kultiviert. Nach 14 Tagen erfolgt die Auswertung. Aus dem Vergleich der Anzahl toter Spinnmilben auf den behandelten zu denjenigen auf unbehandelten Pflanzen wird die prozentuale Reduktion an Population (% Wirkung) bestimmt.

Die Verbindungen der Formel I zeigen eine gute Wirkung gegen Panonychus ulmi in diesem Test.

## Ansprüche

1. N-Benzoyl-N'-[2-fluor-4-(3-chlor-pyridin-2-yloxy)-phenyl]-harnstoffe der Formel I

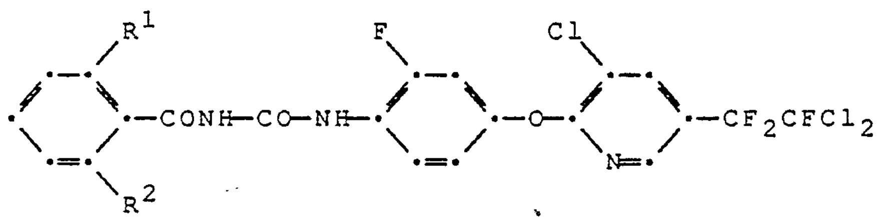

(I),

worin
$R^1$ und $R^2$ beide Fluor oder
$R^1$ Chlor und $R^2$ Wasserstoff bedeuten, sowie deren Salze.

2. N-(2,6-Difluorbenzoyl)-N'-{2-fluor-4-[3-chlor-5-(2,2-dichlor-1,1,2-trifluorethyl)-pyridin-2-yloxy]-phenyl}harnstoff gemäss Anspruch 1.

3. N-(2-Chlorbenzoyl)-N'-{2-fluor-4-[3-chlor-5-(2,2-dichlor-1,1,2-trifluorethyl)-pyridin-2-yloxy]-phenyl}harnstoff gemäss Anspruch 1.

4. Verfahren zur Herstellung der N-Benzoyl-N'-[2-Fluor-4-(pyridin-2-yloxy)phenyl]-harnstoffe der Formel I gemäss Anspruch 1, dadurch gekennzeichnet, dass man die Verbindung der Formel II

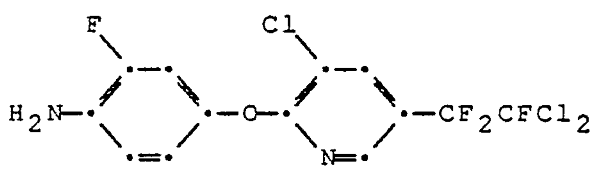

(II)

mit einer Verbindung der Formel III umsetzt

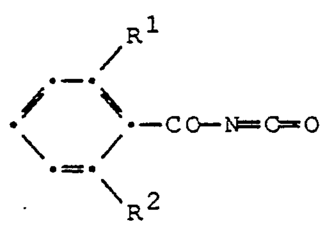

(III),

wobei $R^1$ und $R^2$ die in Anspruch 1 angegebenen Bedeutungen haben.

5. Verfahren zur Herstellung der N-Benzoyl-N′-[2-fluor-4-(pyridin-2-yloxy)-phenyl]-harnstoffe der Formel I gemäss Anspruch 1, dadurch gekennzeichnet, dass man die Verbindung der Formel IV

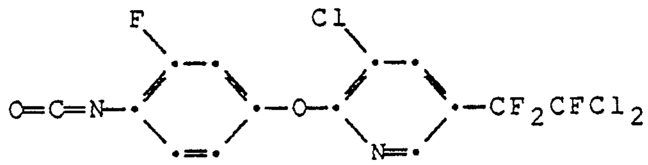

(IV)

mit einer Verbindung der Formel V umsetzt,

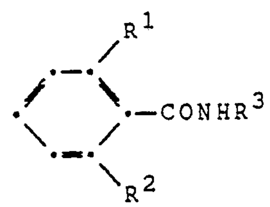

(V),

wobei

$R^1$ und $R^2$ die im Anspruch 1 angegebenen Bedeutungen haben,

$R^3$ Wasserstoff oder einen Rest -COOR$^4$ bedeutet und

$R^4$ für $C_1$-$C_6$Alkyl, Phenyl oder Benzyl

steht.

6. 2-Fluor-4-[3-chlor-5-(2,2-dichlor- 1,1,2-trifluorethyl)pyridin-2-yloxy]anilin der Formel II

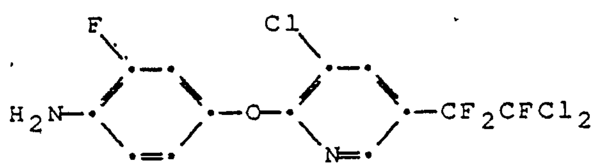

(II).

7. Verfahren zur Herstellung des 2-Fluor-4-[3-chlor-5-(2,2-dichlor-1,1,2-trifluorethyl)-pyridin-2-yloxy]-anilins gemäss Anspruch 6, dadurch gekennzeichnet, dass man 2-Fluor-4-hydroxyanilin in Gegenwart einer Base mit 2,3-Dichlor-5-(2,2-dichlor-1,1,2-trifluorethyl)-pyridin kondensiert.

8. Schädlingsbekämpfungsmittel, welches als aktive Komponente eine Verbindung gemäss Anspruch 1 oder eines ihrer Salze zusammen mit geeigneten Trägern und/oder anderen Zuschlagstoffen enthält.

9. Verwendung einer Verbindung gemäss Anspruch 1 oder eines ihrer Salze zur Bekämpfung von Insekten, Vertretern der Ordnung Akarina und Schnecken.

10. Verwendung gemäss Anspruch 9 zur Bekämpfung larvaler Stadien pflanzenschädigender Insekten.

11. Verwendung gemäss Anspruch 9 als Insekten-Ovizid.

12. Verwendung gemäss Anspruch 9 zur Bekämpfung pflanzenschädigender Spinnmilben.

13. Verfahren zur Bekämpfung von Insekten, Vertretern der Ordnung Akarina und Schnecken, dadurch gekennzeichnet, dass man diese Schädlinge bzw. deren verschiedene Entwicklungsstadien oder deren Aufenthaltsort mit einer pestizid wirksamen Menge einer Verbindung der Formel I gemäss Anspruch 1 oder eines ihrer Salze oder mit einem Mittel enthaltend neben Zusatz- und Trägerstoffen eine pestizid wirksame Menge dieser Verbindung, in Kontakt bringt oder behandelt.

14. Verfahren gemäss Anspruch 13 zur Bekämpfung larvaler Stadien pflanzenschädigender Insekten.

15. Verfahren gemäss Anspruch 13 zur Bekämpfung von Insekten-Eiern.

16. Verfahren gemäss Anspruch 13 zur Bekämpfung pflanzenschädigender Spinnmilben.

Patentansprüche für folgenden Vertragsstaat: ES

1. Verfahren zur Herstellung von N-Benzoyl-N′-[2-fluor-4-(3-chlor-pyridin-2-yloxy)-phenyl]-harnstoffen der Formel I

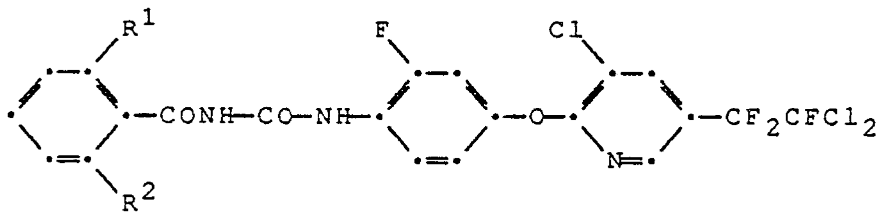 (I),

worin
$R^1$ und $R^2$ beide Fluor oder
$R^1$ Chlor und $R^2$ Wasserstoff bedeuten, sowie deren Salze,
dadurch gekennzeichnet, dass man
a) die Verbindung der Formel II

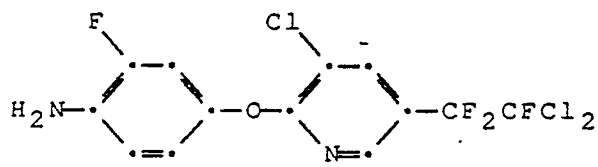 (II)

mit einer Verbindung der Formel III umsetzt

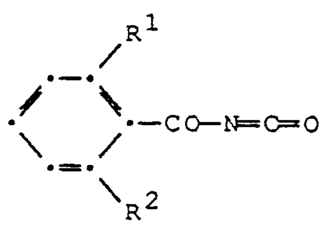 (III),

b) die Verbindung der Formel IV

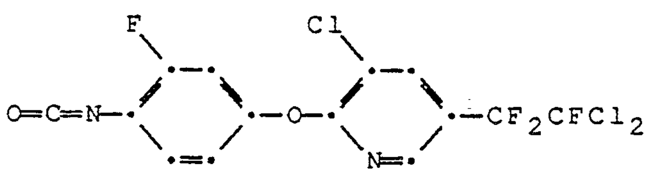 (IV)

mit einer Verbindung der Formel V umsetzt,

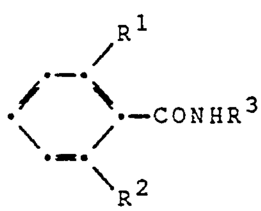 (V),

wobei
$R^1$ und $R^2$ die oben angegebenen Bedeutungen haben,
$R^3$ Wasserstoff oder einen Rest -$COOR^4$ bedeutet und
$R^4$ für $C_1$-$C_6$Alkyl, Phenyl oder Benzyl
steht.

2. Verfahren gemäss Anspruch 1 zur Herstellung von N-(2,6-Difluorbenzoyl)-N′-{2-fluor-4-[3-chlor-5-(2,2-dichlor-1,1,2-trifluorethyl)-pyridin-2-yloxy]-phenyl} harnstoff.

3. Verfahren gemäss Anspruch 1 zur Herstellung von N-(2-Chlorbenzoyl)-N′-{2-fluor-4-[3-chlor-5-(2,2-dichlor-1,1,2-trifluorethyl)-pyridin-2-yloxy]-phenyl} harnstoff.

4. Verfahren zur Herstellung des 2-Fluor-4-[3-chlor-5-(2,2-dichlor-1,1,2-trifluorethyl)-pyridin-2-yloxy]-anilins der Formel II gemäss Anspruch 1, dadurch gekennzeichnet, dass man 2-Fluor-4-hydroxyanilin in Gegenwart einer Base mit 2,3-Dichlor-5-(2,2-dichlor-1,1,2-trifluorethyl)-pyridin kondensiert.

5. Schädlingsbekämpfungsmittel, welches als aktive Komponente eine Verbindung gemäss Anspruch 1 oder eines ihrer Salze zusammen mit geeigneten Trägern und/oder anderen Zuschlagstoffen enthält.

6. Verwendung einer Verbindung gemäss Anspruch 1 oder eines ihrer Salze zur Bekämpfung von Insekten, Vertretern der Ordnung Akarina und Schnecken.

7. Verwendung gemäss Anspruch 6 zur Bekämpfung larvaler Stadien pflanzenschädigender Insekten.

8. Verwendung gemäss Anspruch 6 als Insekten-Ovizid.

9. Verwendung gemäss Anspruch 6 zur Bekämpfung pflanzenschädigender Spinnmilben.

10. Verfahren zur Bekämpfung von Insekten, Vertretern der Ordnung Akarina und Schnecken, dadurch gekennzeichnet, dass man diese Schädlinge bzw. deren verschiedene Entwicklungsstadien oder deren Aufenthaltsort mit einer pestizid wirksamen Menge einer Verbindung der Formel I gemäss Anspruch 1 oder eines ihrer Salze oder mit einem Mittel enthaltend neben Zusatz- und Trägerstoffen eine pestizid wirksame Menge dieser Verbindung, in Kontakt bringt oder behandelt.

11. Verfahren gemäss Anspruch 10 zur Bekämpfung larvaler Stadien pflanzenschädigender Insekten.

12. Verfahren gemäss Anspruch 10 zur Bekämpfung von Insekten-Eiern.

13. Verfahren gemäss Anspruch 10 zur Bekämpfung pflanzenschädigender Spinnmilben.

Europäisches Patentamt

# EUROPÄISCHER RECHERCHENBERICHT

Nummer der Anmeldung

EP 90810338.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | DE - A1 - 3 628 864 (CIBA-GEIGY) * Ansprüche 1,11-15 * -- | 1,4-6, 8,9,13 | C 07 D 213/64<br>A 01 N 47/34 |
| A | US - A - 4 310 530 (NISHIYAMA) * Ansprüche 1,6 * -- | 1,5 | |
| A | EP - A2/A3 - 0 177 455 (CIBA-GEIGY) * Ansprüche 1,9,11,12,14 * ---- | 1,4-6, 8,9,13 | |

| RECHERCHIERTE SACHGEBIETE (Int Cl 5) |
|---|
| C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 26-07-1990 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument